# EUROPEAN PATENT SPECIFICATION

(11) **EP 1 441 586 B1**
(45) Date of publication and mention of the grant of the patent: **21.06.2006**
(21) Application number: 02778703.5
(22) Date of filing: 01.11.2002
(51) Int. Cl.: A01N 1/00

(54) **SYSTEMS AND METHODS FOR FREEZING AND STORING BIOPHARMACUETICAL MATERIAL**
VORRICHTUNG UND VERFAHREN ZUM EINFRIEREN UND ZUR LAGERUNG VON BIOPHARMAZEUTISCHEM MATERIAL
SYSTEMES ET PROCEDES POUR CONGELER ET STOCKER DES MATERIAUX BIOPHARMACEUTIQUES

(30) Priority: 01.11.2001 US 334622 P
(43) Date of publication of application: 04.08.2004
(60) Divisional application: 05110599.7
(73) Proprietor: Integrated Biosystems Inc., Napa, CA 94558 (US)
(72) Inventor: VOUTE, Nicolas, F-13780 Cuges les Pins (FR); LOK, Maxime, 13009 Marseille (FR); LEONARD, Leonidas, Cartwright, Mill Valley, CA 94941-2577 (US); HUGHES, Timothy, Gerard, Benicia, CA 94510 (US); WISNIEWSKI, Richard, San Mateo, CA 94402 (US)
(74) Representative: Buzzi, Franco
(86) International application number: PCT/US2002/035137
(87) International publication number: WO 2003/037083

(56) References cited:
- WO-A-98/23907
- US-A- 4 107 937
- US-A- 4 490 982

## Description

### CROSS-REFERENCE TO RELATED APPLICATIONS

This application claims priority to U.S. Patent Application Serial No. 10/254,036, filed September 23, 2002, and U.S. Provisional Patent Application No. 60/334,622, filed November 1, 2001.

### TECHNICAL FIELD

This invention relates, in general, to biopharmaceutical material, preservation methods and systems, and more particularly to a system and method for freezing, and/or storing biopharmaceutical materials.

### BACKGROUND ART

Preservation of biopharmaceutical materials is important in the manufacture, storage, sale and use of such materials. For example, biopharmaceutical materials are often preserved by freezing between processing steps and during storage. Similarly, biopharmaceutical materials are often frozen during transportation between manufacturing locations.

Currently, preservation of biopharmaceutical material often involves placing a container containing liquid biopharmaceutical material in a cabinet freezer, chest freezer or walk-in freezer and allowing the biopharmaceutical material to freeze. Specifically, the container is often placed on a shelf in the cabinet freezer, chest freezer or walk-in freezer and the biopharmaceutical material is allowed to freeze. These containers may be stainless-steel vessels, plastic bottles or carboys, or plastic flexible containers. They are typically filled with a specified volume to allow for freezing and expansion and then transferred into the freezers at temperatures typically ranging from negative 20 degrees Celsius to negative 70 degrees Celsius or below.

To ensure efficient use of available space inside the freezer, containers are placed alongside one another and sometimes are stacked into an array with varied spatial regularity. Under these conditions, cooling of the biopharmaceutical solution occurs at different rates depending on the exposure of each container to the surrounding cold air, and the extent to which that container is shielded by neighboring containers. For example, containers placed close to the cooling source or those on the outside of an array of containers would be cooled more rapidly than those further away from the cooling source and/or situated at the interior of the array.

In general, adjacent placement of multiple containers in a freezer creates thermal differences from container to container. The freezing rate and product quality then depend on the actual freezer load, space between the containers, and air movement in the freezer. This results in a different thermal history for the contents of the containers depending on their location in a freezer, for example. Also, the use of different containers for individual portions of a single batch of biopharmaceutical material may cause different results for portions of the same batch due to different thermal histories resulting from freezing in a multiple container freezer, particularly if the storage arrangement is haphazard and random. Another consequence of obtaining a range of freezing times is that certain containers may freeze so slowly that the target solute can no longer be captured within the ice phase, but remains in a progressively smaller liquid phase. This phenomenon is referred to as "cyroconcentration." In some cases such cyroconcentration could result in pH change, unfolding, aggregation, or precipitation of the biopharmaceutical product, thus resulting in product loss.

Disposable containers such as plastic flexible containers or other flexible containers often are damaged, leading to loss of the biopharmaceutical material. Particularly, the volumetric expansion of the biopharmaceutical materials during freezing could generate excessive pressure in an over filled bag or in a pocket of occluded liquid adjoining the bag material, possibly leading to rupture or damage to the integrity of the bag. Moreover, handling of such disposable containers, such as plastic flexible containers, during freezing, thawing, or transportation of these containers often result in damage thereof, due, for example, to shock, abrasion, impact, or other mishandling events arising from operator errors or inadequate protection of the flexible containers in use.

Thus, there is a need for systems and methods for freezing, storing, and thawing of biopharmaceutical materials that are controlled, do not result in loss of biopharmaceutical material, but instead create conditions conducive to preserving the biophannaceutical material in a uniform, repeatable fashion in a protected environment.
US-A-4 107 397, which is considered the closest prior art document, discloses a bio-receptacle and holder system for the deep freezing of biological substances, comprising also a sensor for measuring and controlling the cooling applied to the receptacle by a bath of liquefied coolant.

### SUMMARY OF THE INVENTION

The present invention concerns a system for freezing, storing and thawing a biopharmaceutical material as well as a method for freezing, storing and thawing a biopharmaceutical material as claimed in claims 1 and 21, respectively.

The present invention provides, in a first aspect, a system for freezing, thawing, and storing a biophannaceutical material, which includes a flexible container adapted to receive liquid biopharmaceutical material therein for freezing, thawing, and storing. The container includes a first substantially trapezoidal portion attached to a second substantially trapezoidal portion.

The present invention provides, in a second aspect, a system for freezing, thawing, and storing biopharmaceutical material which includes a flexible container, a conduit and a temperature control unit. The flexible container is adapted to receive a liquid biopharmaceutical material therein for freezing and storing. The container may be formed of a first substantially flat sheet of flexible material joined together by a seam with a second substantially flat sheet of flexible material so as to lie substantially flat when empty and wherein the container fully encloses an interior portion for receiving the biopharmaceutical material. Also, the container is configured to form a three-dimensional shape when filled with the biopharmaceutical material wherein the three dimensional shape has a first side and a second side opposite the first side. The conduit is connected to the flexible container to allow the outside of the container to be in fluid communication with the interior portion of such conduit. The temperature control unit includes a first surface and a second surface facing the first surface. Also, the temperature control unit is configured to receive the flexible container therein, when the container is filled with the biopharmaceutical material. The container conforms to the shape of the interior of the temperature control unit and the first side and the second side of the container contact the first surface and the second surface of the temperature control unit. The first and/or second surfaces of the temperature control unit include a heat transfer surface(s).

The present invention provides, in a third aspect, a system for freezing, thawing, and storing a biopharmaceutical material which includes a flexible container and a temperature control unit. The flexible container is adapted to receive liquid biopharmaceutical material therein for freezing, thawing, and storing. Also, the container is configured to conform to a shape of an interior of a temperature control unit, in response to the container being substantially filled with the biopharmaceutical material. The temperature control unit includes at least one heat transfer surface and at least one movable wall adapted to compress the container to inhibit a clearance between the container and the at least one heat transfer surface. Further, the at least one moveable wall may include the at least one heat transfer surface or the at least one moveable wall may provide support for the flexible container without having heat transfer capabilities.

The present invention provides, in a fourth aspect, a system for freezing, thawing, and storing a biopharmaceutical material, which includes a container adapted to receive biopharmaceutical therein for freezing. The container is configured to conform to a shape of an interior of a temperature control unit, when the container is substantially filled with the biopharmaceutical material. Also, the container includes a first portion attached to a second portion wherein the first portion and the second portion are flat. Furthermore, the portions may be substantially trapezoidal portions, substantially triangular portions, substantially rectangular portions, substantially parallelepipedic portions, substantially elliptic portions, substantially semicircular portions, or substantially parabolic portions. The interior of the temperature control unit may have the same shape as the aforementioned containers, when the containers are substantially filled.

The present invention provides, in a fifth aspect, a method for freezing, thawing, and storing a biopharmaceutical material which includes connecting a first flat substantially trapezoidal portion to a second flat substantially trapezoidal portion to form a container adapted to contain the biopharmaceutical material for freezing and adapted to conform to a shape of an interior of a temperature control unit.

The present invention provides, in a sixth aspect, a method for freezing and storing a biopharmaceutical material. The method includes providing a container adapted to contain the biopharmaceutical material for freezing and adapting the container to conform to a shape of an interior of a temperature control unit. The method further includes compressing the container, when it is received in the interior of the temperature control unit, to inhibit a clearance between the container and at least one heat transfer surface of the temperature control unit.

The present invention provides, in a seventh aspect a method for freezing, thawing, and storing a biophaimaceutical material. The method includes joining a first substantially flat sheet of flexible material by a seam with a second substantially flat sheet of flexible material to form a flexible container adapted to receive a biopharmaceutical therein for freezing, thawing, and storing. An interior portion of the container is fully enclosed for receiving the biopharmaceutical material by the joining of the first substantially flat sheet of flexible material with the second substantially flat sheet of flexible material. A three-dimensional shape is formed with the container by filling the container with the biopharmaceutical material wherein the three-dimensional shape has a first side and a second side opposite the first side. A conduit may be connected to the flexible material wherein the outside of the container is in fluid communication with the interior portion via the conduit. The container may be received in a temperature control unit having a first surface and second surface facing the first surface and the shape of the container may conform to an interior of the temperature control unit. The first surface and the second surface of the temperature control unit may contact the first side and the second side of the container wherein the first surface and/or the second surface may include a heat transfer surface.

### BRIEF DESCRIPTION OF THE DRAWINGS

The subject matter which is regarded as the invention is particularly pointed out and distinctly claimed in the claims at the conclusion of the specification. The foregoing and other features, and advantages of the invention will be readily understood from the following detailed description of preferred embodiments taken in conjunction with the accompanying drawings in which:

FIG. 1 is block diagram of a system for freezing, thawing, and storing biopharmaceutical material, in accordance with the present invention;

FIG. 2 is a side cross-sectional view of a temperature control unit of the system of FIG. 1 having a container therein;

FIG. 3 is a side elevational view of a flexible container for thawing, storing, and freezing biopharmaceutical materials useable in the system of Fig. 1 prior to assembly thereof;

FIG. 4 is a top cross-sectional view of the temperature control unit of FIG. 1;

FIG. 5 is a front cross-sectional view of the temperature control unit of FIG. 1;

FIG. 6 is a block diagram of a system for regulating the temperature of a plurality of flexible containers for holding biopharmaceutical material; and

FIG. 7 is a side elevational view of the container of FIG. 3 when substantially filled with biopharmaceutical materials and compressed in the interior of the temperature control unit of the system of FIG. 1.

### DETAILED DESCRIPTION OF ONE

### PREFERRED EMBODIMENT OF THE INVENTION

In accordance with the principles of the present invention, systems and methods of preserving and storing biopharnzaceutical materials are provided.

When processing biopharmaceutical materials such as cells for cryopreservation, for example, if the cells are frozen too quickly, with too high of a water content, the cells may develop intracellular ice crystals. As a result, the cells may rupture and/or become unviable. On the other hand, if the cells are frozen too slowly, the cells are exposed to concentrated solutes over extended period of time, which may also lead to cell damage.

The freezing rate may affect biopharmaceutical material distribution within a frozen volume with nonuniform distribution of biopharmaceutical materials leading to detrimental effects. In an embodiment, control of the freezing rate may be represented as control of the dendritic freezing front velocity, with the dendritic freezing front moving from a cooled wall into a bulk region of the biopharmaceutical material. The freezing rate also affects the final frozen matrix, which may have biopharmaceutical material-protecting or biopharmaceutical material-damaging characteristics. For example, a frozen matrix with biopharmaceutical material embedded into a vitrified portion between dendritic ice crystals may be a biopharmaceutical material -protecting type. Biopharmaceutical material-damaging matrices may take different forms; for example: (1) a very tight cellular ice crystal matrix or (2) an assembly of a very large number of fine ice crystals with product located in very thin layers along the crystal boundaries. The frozen matrix characteristics depends on the ice crystal structure with preferred structure being the dendritic ice crystal structure. Such desirable matrix structure depends primarily on a freezing front velocity with other secondarily important factors being the temperature gradient, composition and concentration of solutes, and geometry of the freezing container.

According to the present invention, maintaining the velocity of a dendritic ice crystal freezing front (hereafter "dendritic freezing front") in a range from approximately 5 millimeters per hour to approximately 250 millimeters per hour, or more preferably in a range from approximately 8 millimeters per hour to approximately 180 millimeters per hour, or most preferably in a range from approximately 10 millimeters per hour to approximately 125 millimeters per hour, provides advantageous cryoprocessing conditions in a wide range of systems and feasible operating margins so that damage to biopharmaceutical materials may be minimized or avoided.

As an example, the following discussion illustrates the relationship between the velocity of dendritic freezing front and the size and spacing of frozen dendrites in the context of freezing of biopharmaceutical materials.

If the velocity of the dendritic freezing front is much lower than approximately 5 millimeters per hour, the dendrites may be small and densely packed within the dendritic freezing front. Consequently, the dendritic freezing front behaves as a solid interface with solutes and biopharmaceutical materials not being integrated into the solid mass, but are being instead rejected and pushed towards the center of a flexible sterile container thus causing severe cryoconcentration in the liquid phase of the biopharmaceutical materials.

As the velocity of dendritic freezing front increases to, but still remains less than approximately 5 millimeters per hour, the dendrites grow somewhat larger in size and more separated, developing into cellular or columnar patterns. In this case, still only a small percentage of the solutes or biopharmaceutical materials become embedded into the solid mass. Instead, most of the solutes and biopharmaceutical materials are pushed forward by the advancing dendritic freezing front and their concentration in the liquid phase of biopharmaceutical material 110 increases. This situation may still result in damage to biopharmaceutical materials.

As the velocity of dendritic freezing front increases to, but still remains less than approximately 5 millimeters per hour, the dendrites grow somewhat larger in size and more separated, developing into cellular or columnar patterns. In this case, still only a small percentage of the solutes or biopharmaceutical materials may become embedded into the solid mass. Instead, most of the solutes and biopharmaceutical materials are pushed forward by the advancing dendritic freezing front and their concentration in the liquid phase of biopharmaceutical material 110 increases. This situation may still result in damage to biopharmaceutical materials.

If the velocity of dendritic freezing front increases beyond approximately 250 millimeters per hour, dendrites start to decrease in size and become more compactly packed, thereby losing the ability to properly embed solutes and particles comprised in biopharmaceutical materials into freezing front.

If the velocity of dendritic freezing front is much higher than approximately 250 millimeters per hour, the resulting solid mass comprises a random, unequalibrated, structure of fine ice crystals. Such rapid cryocooling could be achieved, for example, by supercooling small volumes of biopharmaceutical materials, by freezing biopharmaceutical materials in thin layers, or by submerging small volumes of biopharmaceutical materials into liquid nitrogen or other cryogenic fluid.

For example, in biopharmaceutical materials subjected to supercooling in a liquid phase followed by a rapid ice crystal growth, the velocity of dendritic freezing front may exceed 1000 mm/sec. Such fast dendritic front velocities can create solid masses that comprise biopharmaceutical materials, wherein the solid masses are not formed of equilibrated ice crystals. These non-equilibrated solid masses are prone to ice recrystallization, when dissolution of smaller ice crystals and growth of larger ice crystals may impose excessive mechanical forces on biopharmaceutical materials. Further, biopharmaceutical materials in non-equilibrated solid masses may be distributed between ice crystals in very thin layers on grain boundaries. This produces a large product-ice contact interface area, due to the very large number of small ice crystals, which is detrimental to biopharmaceutical materials.

Inter-dendritic spacing can be regulated by increasing or decreasing the heat flux out of the system (thereby influencing thermal effects and the resulting dendritic freezing front velocities), and by selection and concentration of solutes.

The length of free dendrites may depend in part on the front velocity and on the temperature gradient along the dendrites. The free dendrite may refer to the length of the dendrite sticking into the liquid phase, or, alternatively, to the thickness of a "mushy zone" or a "two-phase zone", e.g., a mixture of dendritic ice crystal needles and liquid phase between them. At the tips of the dendrites, the temperature is close to 0° C, and decreases gradually to match the wall temperature along the dendrite length and the solidified mass away from the front. The temperature of liquid between the dendrites also decreases with nearness to the cold wall. As cryocooling continues, with certain solutes such as salts, the solute concentration reaches a eutectic concentration and temperature.

The solution between the dendrites then solidifies, reaching the complete or substantially complete, or solid, dendritic state. This state is a matrix of the dendritic ice crystals and solidified solutes in a eutectic state between those dendritic ice crystals. Some solutes (for example, carbohydrates) do not form eutectics. Instead they may form a glassy state or crystallize between the dendritic ice crystals. The glassy state may protect a biopharmaceutical product, whereas a crystalline state may have a detrimental effect upon a biopharmaceutical product. Dendritic ice crystals are described further in R. Wisniewski, Developing Large-Scale Cryopreservation Systems for Biopharmaceutical Systems, BioPharm 11(6):50-56 (1998) and R. Wisniewski, Large Scale Cryopreservation of Cells, Cell Components and Biological Solutions, BioPharm 11 (9):42-61 (1998).

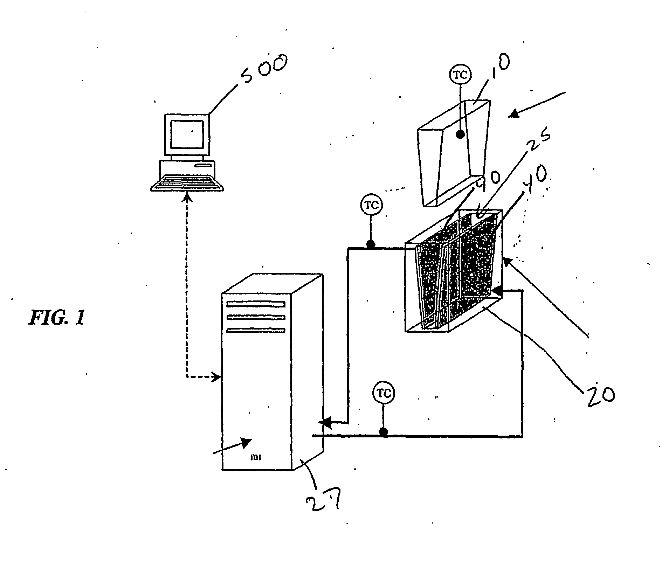

In an exemplary embodiment depicted in FIGS. 1-5, portions of a system for cooling, thawing, preserving and storing biopharmaceutical materials are shown. This system may include a sterile container, such as a flexible container 10, adapted to contain the biopharmaceutical materials and configured to conform to a shape of an interior of a temperature control unit, such as a heat exchanger 20.

Heat exchanger 20 is configured to be operatively coupled to a temperature regulating unit 27 for controlling fluid flow through a conductive medium such as heat transfer plates 40 of heat exchanger 20 to control the temperature of an interior 25 of heat exchanger 20. A controller 500 allows a user to control temperature regulating unit 27 to control heating and/or cooling of the conductive medium, such as plates 40, to cause freezing or thawing, for example, of biopharmaceutical materials in a container, such as flexible container 10, when it is inserted into interior 25 of heat exchanger 20. One example of a heat exchanger is described in co-owned U.S. patent application number 09/905,488 filed July 13, 2001, and co-owned U.S. Patent application No. 09/863,126, filed May 22, 2001. The cooling systems described in the aforementioned applications, and freezing and/or thawing techniques described therein, may be used in conjunction with the systems and methods of freezing and storing biopharmaceutical materials of the present invention. Specifically, the coolers or heat exchangers described in these applications may be configured to incorporate and/or receive the containers for storing biopharmaceutical materials described herein and any associated structures.

For example, temperature regulating unit 27 may control fluid flow through heat transfer plates 20 to control a rate of dendritic freezing front velocity within the biopharmaceutical materials through feedback temperature information regarding biopharmaceutical materials from one or more temperatures sensors (not shown) which may be inserted into container 10 through port 200 or may be connected to, or integral to, plates 20. This feedback loop permits more precise control of heat removal from the biopharmaceutical materials, and facilitates control of the dendritic freezing front velocity to within the recited ranges. Variables such as wall thickness of flexible sterile container 10, thermal resistance between flexible sterile container 10 and plates 20, etc., are automatically taken into account through the feedback loop.

The dendritic freezing front separates biophannaceutical materials present as solid mass from the liquid form of the biopharmaceutical materials, thereby producing a solid-liquid interface in which dendrites are forming. As heat removal from the biopharmaceutical materials continues, the dendritic freezing front advances away from the inner surface of flexible sterile container 10, as additional liquid biopharmaceutical materials freeze into a solid mass. In an embodiment of the present invention, the dendritic freezing front velocity is the velocity with which the dendritic freezing front advances.

In an embodiment, the rate at which heat is removed (i.e., the heat flux) from the biopharmaceutical materials determines the velocity of the dendritic freezing front. Since the temperature gradient between biopharmaceutical materials and plates 20 is correlated with the rate at which heat is removed from the biopharmaceutical materials, the velocity of the dendritic freezing front can be controlled by controlling the temperature of plates 20.

In a preferred embodiment, heat is removed from biopharmaceutical materials at a rate that promotes a substantially uniform advance of the dendritic freezing front within substantially all volume of the biopharmaceutical materials or a substantially constant velocity of the dendritic freezing front. Maintenance of a substantially constant velocity of the dendritic freezing front within flexible sterile container according to an embodiment of this invention is desirable because it provides substantially steady-state conditions for undisturbed dendritic ice crystal growth, independently from the distance to the cooled heat transfer surface within the freezing volume.

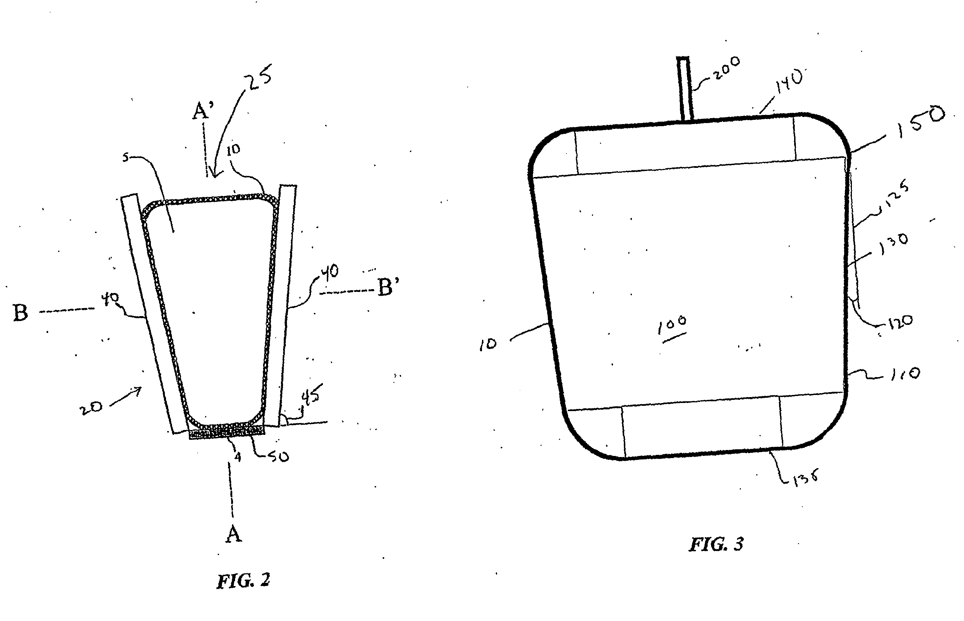

As depicted in FIG. 2, heat transfer plates 40 may be arranged non-parallel relative to one another and may be arranged relative to bottom wall 50 such that interior 25 comprises a tapered space with a smaller distance between heat transfer plates 40 at their bottom ends than at their top ends. This tapered slot provides a larger cross-section at its top extent than its lower extent thus providing a head pressure which also serves to promote contact of flexible container 10 with heat transfer plates 40. Also, the tapered arrangement of plates 40 is favorable since it provides means for directing the volumetric ice expansion in an upward vertical direction. Because the distance between plates 40 increases along a vertical axis of flexible container 10, the time required for solidification fronts (e.g., dendritic freezing fronts) to meet at a mid-point between the plates increases along the vertical axis. Thus, the volumetric ice expansion is not constrained because there is always a liquid or air cavity above. Such an arrangement inhibits uncontrolled volumetric ice expansion, which can place stresses on walls of containers, such as flexible containers, that may result in ruptures of the containers and loss of biopharmaceutical materials.

Flexible container 10 may be formed of a laminated film which includes several layers and may have an interior volume ranging from 0.02-1000 liters, for example. More preferably, flexible container 10 may have a volume between 0.5-250 liters and most preferably, flexible container 10 may have a volume between 0.100-0.5 liters. For example, a biocompatible product-contacting layer may be formed of a low density polyethylene, very low density polyethylene, ethylene vinyl acetate copolymer, polyester, polyamide, polyvinylchloride, polypropylene, polyfluoroethylene, polyvinylidenefluoride, polyurethane, fluoroethylenepropylene, ethylene-vinyl alcohol copolymer, polytetrafluoroethylene, polypropylenes, and copolymers, mixtures or laminates that comprise the above. A gas and water vapor barrier layer may be formed of an ethylene/vinyl alcohol copolymer mixture within a polyamide or an ethylene vinyl acetate copolymer. Further, flexible container 10 may include a layer with high mechanical strength (e.g. a polyamide), and an external layer with insulating effect to heat welding, for example, polyester. The layers may be compatible with cold conditions and may be able to withstand ionization for sterilization purposes. One example of materials useful for formulation of flexible container 10 is described in U.S. patent No. 5,988,442 to Vallot.

Flexible container 10 may be formed by welding the edges of two flat sheets of the materials described above to one another to form a seam and to enclose an interior portion therebetween. For example, two such flat sheets may be formed in a substantially trapezoidal shape thus resulting in the welding of the edges thereof forming a bag of a substantially trapezoidal shape, as best depicted in FIG. 3. For example, a top trapezoidal portion 100 might be welded to a bottom identically sized trapezoidal portion (not shown) at a seam 110 resulting in a flat bag having a trapezoidal shape. Further the trapezoidal portions may be formed such that, when arranged flat one atop the other, an angle 120 between a side 130 of flexible container 10 and an indicator line 125 substantially perpendicular to a bottom side 135 and/or a top side 140 of flexible container 10 may be complimentary to an angle 45 (FIG. 2) between one of heat transfer plates 40 (FIG. 2) and a bottom side 50 (FIG. 2) of heat exchanger 20. For example, angle 45 may be in a range from 0.001 degree to 45 degrees, more preferred being between 0.1 degree and 20 degrees and most preferred being between 0.5 degree and 15 degrees.

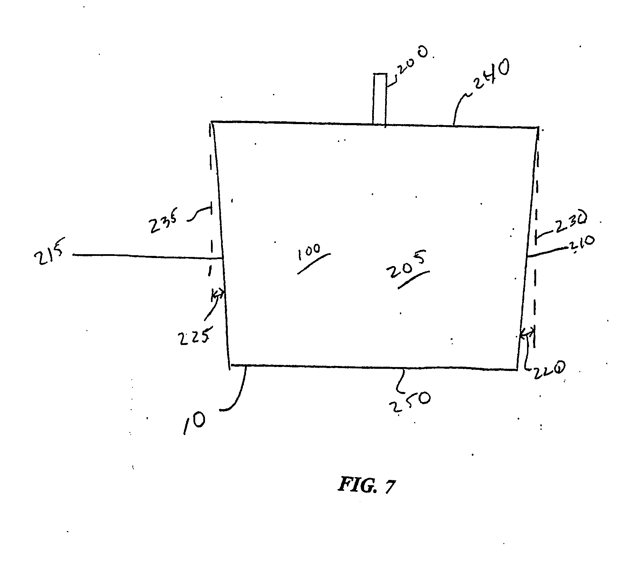

Further, flexible container 10 may form a prismatic shape (e.g., an equilateral prismatic shape) in response to being filled with the biopharmaceutical material and being supported by the tapered slot formed by two non-parallel heat transfer plates 40. Also, when flexible container 10 is substantially filled with a liquid biopharmaceutical material, two opposite sides of flexible container 10 crossed at their mid-point by seam 110 may be trapezoids, e.g. a first trapezoidal side 205 and a second trapezoidal side (not shown), as depicted in FIG. 7. Also, a substantially rectangular lateral side 210 adjacent to first trapezoidal side 205 may be at an angle 220 to an indicator line 230 substantially perpendicular to a top side 240 and/or a bottom side 250 of flexible container 10. A second substantially rectangular lateral side 215 adjacent to first trapezoidal side 205 may be at a second angle 225 relative to a second indicator line 235 substantially perpendicular to top side 240 and/or bottom side 250. Angle 220 and angle 225 may be substantially equivalent to angle 120 formed when flexible container 10 is flat and top trapezoidal portion 100 is arranged flat atop the bottom trapezoidal portion (not shown), as depicted in FIG. 3. Moreover, a bag adapted to receive a biopharmaceutical material could be formed of two similarly sized flat sheets of other shapes welded to each other, e.g., rectangular, triangular, parallelepipedic, elliptic, semicircular, or parabolic shaped sheets. For example, a bag formed of two similarly sized flat rectangular sheets would have two opposite rectangular sides, when substantially filled with the biopharmaceutical material and supported by two parallel heat transfer plates 40.

The formation of flexible container 10 in a substantially prismatic shape, when substantially filled with biopharmaceutical material, for example, as described above, allows flexible container 10 to be received in, and substantially conform to the shape of interior 25 of heat exchanger 20, as depicted in FIGS. 1-2 and 4-5. For example, seam 110 (FIG. 3) connecting top side 100 with bottom side (not shown) of flexible container 10 may be received in interior 25 such that seam 110 abuts one of side walls 60 of heat exchanger 40. Further, in response to flexible container 10 being filled with the biopharmaceutical material, seam 110 may be located in a plane substantially perpendicular to one or both of side walls 60. This configuration allows flexible container 10 to substantially contact heat transfer plates 40. Also, side walls 60 may include depressions on interior sides thereof to receive seams 110. Such depressions also promote contact between flexible container 10 and heat transfer plates 40. Such contact is desirable since air between flexible container 10 and heat transfer plates 40 may act as an insulator therebetween which could inhibit or delay cooling or heating of the biopharmaceutical material in flexible container 10.

Further, the formation of flexible container 10 using two trapezoidal portions allows it to substantially conform to the tapered slot of interior 25 and to inhibit deformation of the side ends of flexible container 10, when flexible container 10 is filled with the biopharmaceutical material and received in interior 25. Tapered or prismatic shaped flexible containers may be preferred to square or rectangular flat flexible containers having rounded corners wherein such rounded corners may not contact heat transfer plates, such as heat transfer plates 40, when received in a device such as heat exchanger 20. Such rounded corners may result in a low ratio of contact surface area to volume. Thus, less direct contact between flexible container 10 and the heat transfer plates and uneven heating or cooling of the biopharmaceutical material held in these flexible containers may result. Further, heating or cooling of the biopharmaceutical material in such square or rectangular flexible containers may be less efficient than flexible container 10 due to the greater contact of flexible container 10 with heat transfer plates 40. Specifically, pockets of air between a bag and heat transfer plates result in less efficient heat transfer since such heat transfer is done by natural convection instead of conduction. The heat transfer plates must cool or heat the air which then cools or heats the bag and biopharmaceutical material as opposed to the heat transfer plates directly cooling the bag and the biopharmaceutical material contained therein. It is thus desirable for the bag to be in direct contact with the heat transfer plates to promote conductive heat transfer, which is more efficient in this case than convective heat transfer of the air, bag, and biopharmaceutical materials. Further, the formation of flexible container 10 to conform to the tapered slot of interior 25 promotes such conductive heat transfer.

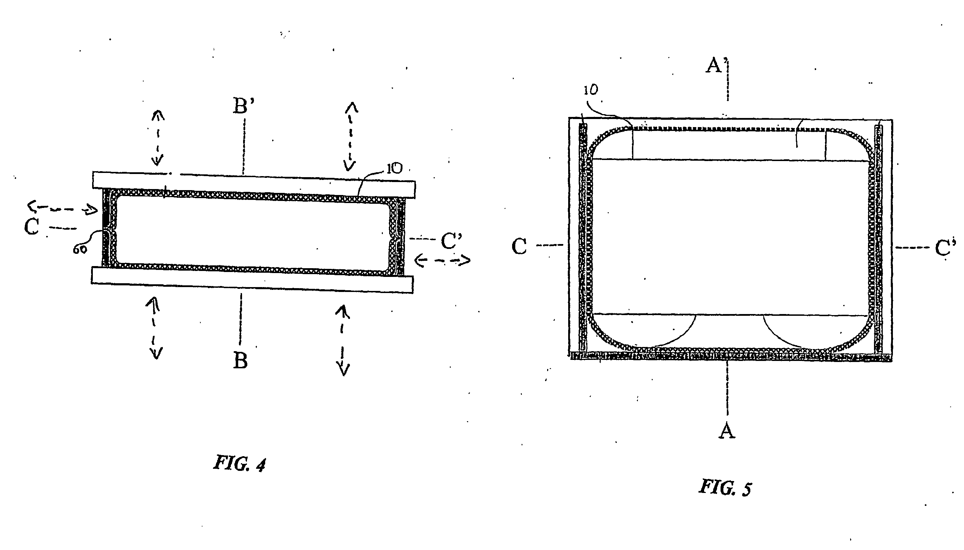

In an alternate embodiment and as shown in FIG. 4, one or both of side walls 60 of heat exchanger 20 may be moveable. Specifically, walls 60 may be moveable toward or away from one another between heat transfer plates 40. The movability of one or more of side walls 60 allows flexible container 10 to be compressed. The compressing promotes contact of flexible container 10 with heat transfer plates 40 while inhibiting any space or clearance between flexible container 10 and heat transfer plates 40. Specifically, such compression may force the biopharmaceutical material or other liquid in flexible container 10 to occupy any voids between heat transfer plates 40 and flexible container 10. For example, such pressure may cause the biopharmaceutical material to occupy such voids or be forced up due to the tapered slot shape of interior 25. Alternatively, side walls 60 may be fixed, whereas heat transfer plates 40 may be moveable towards or away from one another. Also, the required compression may be achieved by one of side walls 60 and/or one of heat transfer plates 40 being moveable. Also, it will be understood by those skilled in the art that side walls 60 may be maintained at particular positions relative to one another and relative to heat transfer plates 40 via pins, notches, or grooves in heat transfer plates 40 or bottom side 50 of heat exchanger 20. Side walls 60 and/or heat transfer plates 40 may be moveably, fixedly, and/or releasably attached to each other and/or bottom wall 50. Further, the ability of side walls 60 and/or heat transfer plates 40 to move could facilitate removal of flexible container 10 received therein. For example, by retracting walls 60 and/or plates 40 removal of frozen biopharmaceutical material held in heat exchanger 20 may be facilitated. Also, it will also be understood by those skilled in the art that side walls 60 and/or bottom 50 could also be adapted to provide cooling and/or heating to flexible container 10 via circulation of heat transfer fluids in coils therein or other means, as will be understood by those skilled in the art.
Compression of flexible container 10 by one or more of side walls 60 and/or heat transfer plates 40 may cause flexible container 10 to form the prismatic shape described above, for example, thus promoting contact between flexible container 10 and heat transfer plates 40. Moreover, the shape of side walls 60 may match profiles of flexible flexible containers of other shapes when compressed. Also, side walls 60 and/or heat transfer plates 40 may be formed of a high thermal conductivity material, for example, stainless steel or aluminum.

As depicted in FIG. 3, flexible container 10 may include an inlet port 200 to allow biopharmaceutical materials to be inserted into the interior (not shown) of flexible container 10 and to be removed therefrom. Inlet port 200 may include a conduit or tube, for example, which may be integral to flexible container 10 or which may be connected to inlet port 200 using a fitting placed within the inlet port. Fittings such as those described in U.S. Patent No. 6,186,932 may be used for the connection of such tubes. Also, fittings which can maintain the sterility of the contents of the container or bag may preferably be used. The fittings may be configured in different shapes, such as straight fittings and/or angled fittings including ninety (90) degree elbows, if desired. Inlet port 200 may include a filter (not shown) to filter any biopharmaceutical material. Flexible container 10 may also be emptied by turning flexible container 10 upside down and allowing the contents to drain. Also, a rigid or semi rigid holder (not shown) may be attached to flexible container 10 to facilitate carrying and/or storage of flexible container 10.

Moreover, although the container is described herein as a flexible container or bag, the container may be made of a semi-rigid material. Such material may be used to construct a container which is shaped to conform to the interior of heat exchanger 20. Preferably, the container whether formed of a flexible or semi-rigid material, includes surfaces which contact the interior surfaces of a heat exchanger 20 so that there is direct contact between the cooled (or heated in a thawing process) surfaces of heat exchanger 20 and the outer surfaces of the container containing biopharmaceutical materials.

After the biopharmaceutical material is frozen (e.g., to negative 20EC or below) or its temperature otherwise regulated in flexible container 10 in a temperature control unit, such as heat exchanger 20, flexible container 10 may be removed therefrom and placed in a freezer for storage or the biopharmaceutical material may be removed from flexible container 10 and utilized.

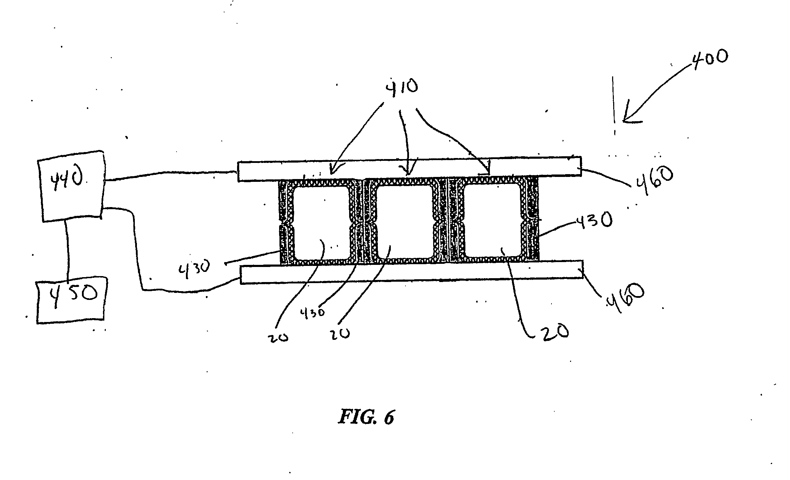

In a further embodiment of the present invention, a heat exchanger 400 may include a plurality of receiving interior portions 410 for receiving a plurality of flexible container 10 adapted to contain biopharmaceutical material, as depicted in FIG. 6. Each receiving interior portion 410 may include a plurality of moveable walls 430 for compressing flexible container 10. Heat exchanger 400 also may include one or more heat transfer plates 460 for regulating a temperature of flexible container 10 and therefore the contents thereof. Heat exchanger 400 is coupled to a temperature regulating unit 440 for regulating temperatures of plates 460 wherein temperature regulating unit 440 is controlled by a controller 450, programmable by a user.

A typical process of preserving biopharmaceutical material is described as follows. Liquid biopharmaceutical material is inserted through port 200 into flexible container 10. Flexible container 10 is then inserted into heat exchanger 20, as shown in FIGS. 1, 2, 4 and 5, where the biopharmaceutical contents are cooled and frozen (e.g., at a sub-zero temperature) in a controlled manner such that the freeze rate is controlled within upper and lower limits, as described in U.S. Patent Application 09/905,485 for example, to prevent or inhibit cryoconcentration of the biopharmaceutical material, thereby preventing undesirable degradation of the biopharmaceutical material. Alternatively, flexible container 10 may be inserted into heat exchanger 20 before the biopharmaceutical material is inserted into flexible container 10. After the biopharmaceutical material in flexible container 10 is frozen, flexible container 10 may be removed from heat exchanger 20 and placed in a large freezer, for example, a walk-in freezer having an interior air temperature of about negative 20EC or lower, as is typically present in pharmaceutical production plants or large medical institutions (e.g., hospitals). The biopharmaceutical material may be removed from the walk-in freezer and inserted in a temperature control unit, such as heat exchanger 20, to be thawed, when it is desired to utilize or further process the biopharmaceutical material. It will be further understood by those skilled in the art that modifications may be made to the specific examples described herein and the steps for performing the method for preserving the biopharmaceutical material.

Also, it will be understood to one skilled in the art that the flexible containers described herein may be adapted for use in vessels, containers, temperature control units, and/or heat exchangers of various shapes or sizes. For example, the flexible containers could be formed in triangular, conical, cylindrical, pyramidal frustum or conical frustum shapes. Further, the temperature control units, e.g., heat exchanger 20, may be formed to have interior portions of various shapes which may be adapted to receive containers or flexible containers of various shapes or sizes. Also, temperature control units of various types may be utilized in addition to heat exchanger 20. For example, blast freezing units might also be utilized to control the temperature of the contents of flexible containers such as flexible container 10. Additionally, cryogenic fluid may be sprayed on flexible container 10 for cooling or warm fluid may be sprayed thereon for heating thereof. Moreover, the temperature control units may be of various shapes or sizes to accommodate containers or flexible containers of various shapes or sizes. The temperature control unit could also be adapted to heat and/or cool interior portions thereof to various temperatures. Also, the temperature control units could include any number of movable walls to compress flexible containers held therein. Furthermore, these flexible containers, containers, temperature control units, blast freezing units, and/or heat exchangers may be adapted for utilization with materials other than biopharmaceutical materials.

Further, although preferred embodiments have been depicted and described in detail herein, it will be apparent to those skilled in the relevant art that various modifications, additions, substitutions and the like can be made without departing from the scope of the invention as defined in the following claims.

## Claims

1. A system for freezing, thawing, and storing a biopharmaceutical material, said system comprising:
a flexible container adapted to receive biopharmaceutical material therein for freezing, thawing, and storing said container comprising a first substantially trapezoidal portion attached to a second substantially trapezoidal portion.

2. The system of claim 1 further comprising a temperature control unit, and wherein said container is configured to conform to a shape of an interior of said temperature control unit in response to said container being substantially filled with the biopharmaceutical material.

3. The system of claim 2 wherein said first portion and said second portion are connected to each other at a seam and wherein said container is adapted to be longitudinally received in said temperature control unit to cause said seam to be located in a plane substantially perpendicular to at least one lateral wall of said temperature control unit.

4. The system of claim 1 wherein a first angle of a lateral side of said container relative to an indicator line substantially perpendicular to a bottom side of said container comprises a first dimension and a second angle of a wall of a temperature control unit relative to a bottom of said temperature control unit comprises a second dimension, and wherein said first dimension is substantially complimentary to said second dimension.

5. The system of claim 4 wherein a third angle of a second lateral side of said container relative to a second indicator line substantially parallel to said indicator line comprises a third dimension and wherein said third dimension is substantially equivalent to said first dimension.

6. The system of claim 4 wherein said wall is a first wall and said temperature control unit comprises a second wall opposite said first wall, a plurality of heat transfer surfaces, a third wall, and a fourth wall, and wherein said third wall and said fourth wall are adapted to move relative to each other to compress said container to cause said container to inhibit a clearance between said container and said plurality of heat transfer surfaces.

7. The system of claim 1 wherein said container is adapted to comprise a prismatic shape in response to being substantially filled with the biopharmaceutical material and being received in an interior of a temperature control unit.

8. The system of claim 1 further comprising a temperature control unit adapted to receive said container, said temperature control unit comprising a wall, wherein said first portion and said second portion are connected to each other at a seam and wherein said wall comprises a depression to receive said seam to allow said container to substantially conform to a shape of an interior of said temperature control unit.

9. The system of claim 1 wherein said container comprises a sterile bag adapted to contain the biopharmaceutical material and wherein said bag is adapted to substantially conform to a shape of an interior of a temperature control unit.

10. The system of claim 1 wherein said container is adapted to contain the biopharmaceutical material at least one of before, during, and after a freezing of the biopharmaceutical material, when the biopharmaceutical material is received in said container.

11. The system of claim 1 wherein said container is adapted to contain the biopharmaceutical material at least one of before, during, and after a thawing of the biopharmaceutical material, when the biopharmaceutical material is received in said container.

12. The system of claim 1 further comprising a temperature control unit adapted to receive said container and adapted to control a temperature of the biopharmaceutical material when the biopharmaceutical material is contained in said container in an interior of said temperature control unit

13. The system of claim 12 further comprising a controller for controlling said temperature control unit wherein said controller is controllable by a user.

14. A method for freezing, thawing, and storing a biophaimaceutical material, the method comprising:
connecting a first flat substantially trapezoidal portion to a second flat substantially trapezoidal portion to form a container adapted to contain the biopharmaceutical material and adapted to conform to a shape of an interior of a temperature control unit

15. The method of claim 14 further comprising locating the container in an interior of the temperature control unit and compressing the container to inhibit a clearance between the container and at least one heat transfer surface of the temperature control unit.

16. The method of claim 15 further comprising causing the container to form a prismatic shape by the compressing.

17. The method of claim 16 further comprising causing the compressing by moving at least one wall of the temperature control unit.

18. The method of claim 14 further comprising at least one of freezing and thawing the biopharmaceutical material in the container in the interior of the temperature control unit.

## Patentansprüche

1. System zum Einfrieren, Auftauen und Speichern eines biopharmazeutischen Materials, wobei das System aufweist:
einen nachgiebigen Behälter, der daran angepasst ist, biopharmazeutisches Material darin aufzunehmen zum Einfrieren, Auftauen und Speichern, wobei der Behälter einen ersten, im Wesentlichen trapezoiden Teil aufweist, der angebracht ist an einem im Wesentlichen zweiten trapezoiden Teil.

2. System nach Anspruch 1, weiter aufweisend eine Temperatursteuerungseinheit, und wobei der Behälter konfiguriert ist, sich einer Form eines Inneren der Temperatursteuerungseinheit anzupassen als Reaktion darauf, dass der Behälter im Wesentlichen mit dem biopharmazeutischen Material gefüllt ist.

3. System nach Anspruch 2, wobei der erste Teil und der zweite Teil miteinander an einer Naht verbunden sind und wobei der Behälter daran angepasst ist, longitudinal in der Temperatursteuerungseinheit aufgenommen zu werden, um zu bewirken, dass die Naht in einer Ebene im Wesentlichen senkrecht zu wenigstens einer lateralen Wand der Temperatursteuerungseinheit angeordnet ist.

4. System nach Anspruch 1, wobei ein erster Winkel einer ersten Seite des Behälters relativ zu einer Anzeigelinie im Wesentlichen senkrecht zur Bodenseite des Behälters eine erste Abmessung aufweist, und ein zweiter Winkel einer Wand einer Temperatursteuerungseinheit relativ zu einem Boden der Temperatursteuerungseinheit eine zweite Abmessung aufweist, und worin die erste Abmessung im Wesentlichen komplimentär zur zweiten Abmessung ist.

5. System nach Anspruch 4, wobei ein dritter Winkel einer zweiten lateralen Seite des Behälters relativ zu einer zweiten Anzeigelinie im Wesentlichen parallel zur Anzeigelinie eine dritte Abmessung aufweist und wobei die dritte Abmessung im Wesentlichen äquivalent zur ersten Abmessung ist.

6. System nach Anspruch 4, wobei die Wand eine erste Wand ist und die Temperatursteuerungseinheit eine zweite Wand aufweist gegenüber der ersten Wand, eine Vielzahl von Wärmeübertragungsoberflächen, eine dritte Wand und eine vierte Wand, wobei die dritte und die vierte Wand daran angepasst sind, sich relativ zueinander zu bewegen, um den Behälter zu komprimieren, um zu bewirken, dass ein Abstand zwischen dem Behälter und der Vielzahl von Wärmeübertragungsüberflächen verhindert wird.

7. System nach Anspruch 1, wobei der Behälter daran angepasst ist, eine prismatische Gestalt aufzuweisen als Reaktion darauf im Wesentlichen mit dem biopharmazeutischen Material befüllt zu sein und aufgenommen in einem Inneren einer Temperatursteuerungseinheit.

8. System nach Anspruch 1, weiter aufweisend eine Temperatursteuerungseinheit, die daran angepasst ist, den Behälter aufzunehmen, die Temperatursteuerungseinheit eine Wand aufweist, wobei der erste Teil und der zweite Teil miteinander verbunden sind in einer Naht und wobei die Wand eine Vertiefung aufweist, um die Naht aufzunehmen, um es dem Behälter zu ermöglichen, dass er sich im Wesentlichen der Gestalt des Inneren der Temperatursteuerungseinheit anpasst.

9. System nach Anspruch 1, wobei der Behälter eine sterile Tasche aufweist, die daran angepasst ist das biopharmazeutische Material zu enthalten und worin die Tasche daran angepasst ist im wesentlichen mit einer Form eines inneren einer Temperatursteuerungseinheit überein zu stimmen

10. System nach Anspruch 1, wobei der Behälter daran angepasst ist, das biopharmazeutische Material wenigstens bei einem vor, während und nach dem Gefrieren des biopharmazeutischen Materials zu enthalten, wenn das biopharmazeutische Material in den Behälter aufgenommen wird.

11. System nach Anspruch 1, wobei der Behälter daran angepasst ist, das biopharmazeutische Material wenigstens aus einem vor, während und nach dem Auftauen des biopharmazeutischen Materials zu enthalten, wenn das biopharmazeutische Material in den Behälter aufgenommen wird.

12. System nach Anspruch 1, weiter aufweisend eine Temperatursteuerungseinheit, die daran angepasst ist, den Behälter aufzunehmen, daran angepasst, eine Temperatur des biopharmazeutischen Materials zu steuern, wenn das biopharmazeutische Material in dem Behälter enthalten ist in einem Inneren der Temperatursteuerungseinheit.

13. System nach Anspruch 12, weiter aufweisend eine Steuerung zur Steuerung der Temperatursteuerungseinheit, wobei die Steuerung durch einen Anwender steuerbar ist.

14. Verfahren zum Einfrieren, Auftauen und Speichern eines biopharmazeutischen Materials, das Verfahren aufweisend:
Verbinden eines ersten flachen, im Wesentlichen trapezoiden Teils mit einem zweiten flachen, im Wesentlichen trapezoiden Teil, um einen Behälter zu bilden, der daran angepasst ist, das biopharmazeutische Material zu enthalten und daran angepasst ist, einer Gestalt eines Inneren einer Temperatursteuerungseinheit zu entsprechen.

15. Verfahren nach Anspruch 14, weiter aufweisend Anordnen des Behälters in einem Inneren der Temperatursteuerungseinheit und Komprimieren des Behälters, um zu verhindern, dass sich ein Abstand zwischen dem Behälter und wenigstens einer Wärmeübertragungsoberfläche der Temperatursteuerungseinheit bildet.

16. Verfahren nach Anspruch 15, weiter aufweisend Bewirken, dass der Behälter eine prismatische Gestalt durch Komprimierung annimmt.

17. Verfahren nach Anspruch 16, weiter aufweisend Bewirken der Kompression durch Bewegung wenigstens einer Wand der Temperatursteuerungseinheit.

18. Verfahren nach Anspruch 14, weiter aufweisend wenigstens eines aus Einfrieren und Auftauen des biopharmazeutischen Materials im Behälter im Inneren der Temperatursteuerungseinheit.

## Revendications

1. Système pour congeler, décongeler et conserver un matériau biopharmaceutique, ledit système comprenant :
un récipient souple adapté pour recevoir un matériau biopharmaceutique dans celui-ci pour congeler, décongeler et conserver ledit récipient comprenant une première partie sensiblement trapézoïdale reliée à une seconde partie sensiblement trapézoïdale.

2. Système selon la revendication 1 comprenant en outre une unité de commande de la température, et dans lequel ledit récipient est configuré pour épouser une forme d'un intérieur de ladite unité de commande de la température en réponse au remplissage dudit récipient avec le matériau biopharmaceutique.

3. Système selon la revendication 2 dans lequel ladite première partie et ladite seconde partie sont reliées l'une à l'autre au niveau d'une soudure et dans lequel ledit récipient est adapté pour être reçu longitudinalement dans ladite unité de commande de la température de sorte que ladite soudure se trouve dans un plan sensiblement perpendiculaire à au moins une paroi latérale de ladite unité de commande de la température.

4. Système selon la revendication 1 dans lequel un premier angle formé par un côté latéral dudit récipient et une ligne indicatrice sensiblement perpendiculaire à un côté inférieur dudit récipient comprend une première dimension et un deuxième angle formé par une paroi d'une unité de commande de la température et un fond de ladite unité de commande de la température comprend une deuxième dimension, et dans lequel ladite première dimension est sensiblement complémentaire de ladite deuxième dimension.

5. Système selon la revendication 4 dans lequel un troisième angle formé par un deuxième côté latéral dudit récipient et une seconde ligne indicatrice sensiblement parallèle à ladite ligne indicatrice comprend une troisième dimension et dans lequel ladite troisième dimension est sensiblement équivalente à ladite première dimension.

6. Système selon la revendication 4 dans lequel ladite paroi est une première paroi et ladite unité de commande de la température comprend une deuxième paroi opposée à ladite première paroi, une pluralité de surfaces de transfert de la chaleur, une troisième paroi et une quatrième paroi, et dans lequel ladite troisième paroi et ladite quatrième paroi sont adaptées pour se déplacer l'une par rapport à l'autre pour comprimer ledit récipient pour faire que ledit récipient comble un espace entre ledit récipient et ladite pluralité de surfaces de transfert de la chaleur.

7. Système selon la revendication 1 dans lequel ledit récipient est adapté pour comprendre une forme prismatique lorsqu'il est sensiblement rempli du matériau biopharmaceutique et lorsqu'il est reçu dans un intérieur d'une unité de commande de la température.

8. Système selon la revendication 1 comprenant en outre une unité de commande de la température adaptée pour recevoir ledit récipient, ladite unité de commande de la température comprenant une paroi, ladite première partie et ladite seconde partie étant reliées l'une à l'autre au niveau d'une soudure et dans lequel ladite paroi comprend un creux pour recevoir ladite soudure pour permettre audit récipient d'épouser sensiblement une forme d'un intérieur de ladite unité de commande de la température.

9. Système selon la revendication 1 dans lequel ledit récipient comprend une poche stérile adaptée pour contenir le matériau biopharmaceutique et dans lequel ladite poche est adaptée pour épouser sensiblement une forme d'un intérieur d'une unité de commande de la température.

10. Système selon la revendication 1 dans lequel ledit récipient est adapté pour contenir le matériau biopharmaceutique au moins avant, pendant ou après une congélation du matériau biopharmaceutique, lorsque le matériau biopharmaceutique est reçu dans ledit récipient.

11. Système selon la revendication 1 dans lequel ledit récipient est adapté pour contenir le matériau biopharmaceutique au moins avant, pendant ou après une décongélation du matériau biopharmaceutique, lorsque le matériau biopharmaceutique est reçu dans ledit récipient.

12. Système selon la revendication 1 comprenant en outre une unité de commande de la température adaptée pour recevoir ledit récipient et adaptée pour commander la température du matériau biopharmaceutique lorsque le matériau biopharmaceutique est contenu dans ledit récipient dans un intérieur de ladite unité de commande de la température.

13. Système selon la revendication 12 comprenant en outre un dispositif de commande pour commander ladite unité de commande de la température dans lequel ledit dispositif de commande peut être commandé par un utilisateur.

14. Procédé pour congeler, décongeler et conserver un matériau biopharmaceutique, le procédé comprenant les étapes suivantes :
relier une première partie plate sensiblement trapézoïdale à une seconde partie plate sensiblement trapézoïdale pour former un récipient adapté pour contenir le matériau biopharmaceutique et adapté pour épouser une forme d'un intérieur d'une unité de commande de la température.

15. Procédé selon la revendication 14 comprenant en outre les étapes de positionnement du récipient dans un intérieur de l'unité de commande de la température et de compression du récipient pour combler un espace entre le récipient et au moins une surface de transfert de chaleur de l'unité de commande de la température.

16. Procédé selon la revendication 15 comprenant en outre l'étape consistant à faire prendre au récipient une forme prismatique par compression.

17. Procédé selon la revendication 16 comprenant en outre l'étape de réalisation de la compression en déplaçant au moins une paroi de l'unité de commande de la température.

18. Procédé selon la revendication 14 comprenant en outre au moins la congélation ou la décongélation du matériau biopharmaceutique dans le récipient à l'intérieur de l'unité de commande de la température.
